# FASCICULE DE BREVET EUROPEEN

(11) **EP 3 406 702 B1**
(45) Date de publication et mention de la délivrance du brevet: **08.04.2020**
(21) Numéro de dépôt: 18174205.7
(22) Date de dépôt: 24.05.2018
(51) Int. Cl.: C12M 1/107, E21F 5/02

(54) **SYSTEME ET PROCEDE DE PRODUCTION DE BIOGAZ**
SYSTEM UND VERFAHREN ZUR HERSTELLUNG VON BIOGASEN
SYSTEM AND METHOD FOR PRODUCING BIOGAS

(30) Priorité: 24.05.2017 FR 1754630
(43) Date de publication de la demande: 28.11.2018
(73) Titulaire: IPSB, 77210 Avon (FR)
(72) Inventeur: LAUNAY, Franck, 77120 AVON (FR); BOUZRARA, Hazem, 77120 AVON (FR)
(74) Mandataire: den Braber, Gérard Paul

(56) Documents cités:
- CN-Y- 201 288 183
- DE-A1-102010 015 063
- US-A1- 2014 024 108
- WANG FAHUI ET AL: "Suppression of methane/air explosion in pipeline by water mist", JOURNAL OF LOSS PREVENTION IN THE PROCESS INDUSTRIES, vol. 49, 8 février 2017 (2017-02-08), pages 791-796, XP085199697, ISSN: 0950-4230, DOI: 10.1016/J.JLP.2017.02.005

## Description

### DOMAINE TECHNIQUE

Un aspect de l'invention concerne un système de production de biogaz. Le biogaz produit peut être valorisable en tant que biocarburant, par exemple en liquéfiant le biogaz. Un autre aspect de l'invention concerne un procédé de production de biogaz.

### ETAT DE LA TECHNIQUE ANTERIEURE

Un biogaz est typiquement produit dans un bioréacteur, notamment un bioréacteur de type fermenteur. Il existe de nombreuses bio-réactions mettant en œuvre des biocatalyseurs et/ou des microorganismes pouvant générer des gaz inflammables tel que l'Hydrogène (H₂), Isobutène, Méthane, ou d'autres molécules gazeuses d'intérêt dans un bioréacteur. Ces gaz se retrouvent pour tout ou partie dans un ciel gazeux situé au-dessus d'un milieu réactionnel sous forme, par exemple, d'un moût de fermentation. Ce ciel gazeux est susceptible, en fonction du type de bio-réaction développée, de contenir plus ou moins d'oxygène, qui est un comburant. Dans certaines conditions, le ciel gazeux, peut être considéré comme une atmosphère explosive.

Il existe donc un risque d'explosion dans un bioréacteur opérationnel. En effet, le risque d'explosion d'un bioréacteur existe suite à une inflammation du ciel gazeux du bioréacteur qui, à un moment donné, réunit différentes conditions requises pour qu'il y ait explosion, à savoir : le confinement, la présence d'un carburant, la présence d'un comburant; la possibilité d'avoir une source d'ignition, et se trouver dans les limites d'explosivité.

Il convient de produire du biogaz dans des conditions de sécurité satisfaisantes. La prise en compte du risque d'explosion dans le ciel gazeux du bioréacteur dans sa conception intrinsèque peut conduire à des coûts importants. Cela peut freiner le développement de la production du biogaz. En effet, si un bioréacteur doit être dimensionné pour qu'il résiste à une explosion thermique, cela peut avoir pour effet d'oblitérer considérablement la rentabilité économique d'un projet de production de biogaz.

La publication brevet US 2014/024108 décrit un appareil pour la production d'un biogaz par digestion anaérobie de matière organique. L'appareil comprend une chambre de digesteur définissant une zone de génération de gaz et une zone de collecte de gaz. Une ou plusieurs buses sont agencées pour pulvériser un liquide de refroidissement de gaz dans le gaz logé dans la zone de collecte de gaz de manière à refroidir le gaz.

### EXPOSE DE L'INVENTION

Il existe un besoin pour une solution économique permettant de réduire, voire même éliminer, le risque d'explosion dans un bioréacteur.

Selon un aspect de l'invention, un brouillard de liquide non-inflammable est généré dans le ciel gazeux du bioréacteur par brumisation. Le brouillard de liquide non-inflammable comprend des gouttelettes ayant un diamètre nominal inférieur à 1 mm de sorte que brouillard de liquide non-inflammable est apte à absorber un rayonnement thermique et à piéger de l'oxygène. Le liquide qui constitue le brouillard peut être, par exemple, de l'eau ou être à base de l'eau comme, par exemple, un mélange d'eau et un ou plusieurs additifs, tel qu'un anti-mousse, ou un moût fermentaire recyclé, qui peut être brut ou épuré.

Le brouillard de liquide permet de sortir du domaine d'explosivité du ciel gazeux et ainsi réduire, voire éliminer, le risque d'explosion grâce à au moins un des effets suivants.

L'absorption du rayonnement thermique par le brouillard de liquide diminue le risque d'ignition du ciel gazeux par échauffement. Un échauffement peut être d'origine externe, par exemple, un feu à proximité du fermenteur. Un échauffement peut aussi être d'origine interne, par exemple l'échauffement d'une garniture, le frottement d'une pièce contre une autre pièce.

La réduction, voire l'élimination, des sources d'ignition dans le ciel gazeux peut aussi se faire par le maintien d'une hygrométrie relativement élevée dans l'atmosphère explosive. Une ignition peut avoir lieu dans le brouillard constitué, mais nécessite un apport d'énergie plus important qu'en l'absence de brouillard.

Le brouillard de liquide peut aussi empêcher un transfert d'énergie de proche en proche par atténuation des phénomènes de radiation. Par exemple, l'énergie développée lors d'un début d'explosion sera absorbée pour l'échauffement des gouttelettes de liquide, par exemple, des gouttelettes d'eau.

Le brouillard de liquide permet un refroidissement des parois internes du bioréacteur dans sa partie supérieure. Cela diminue le risque d'éclatement mécanique par compression de gaz. Ce refroidissement permet aussi que ces parois internes n'atteignent pas une température supérieure à, par exemple, 80% de la température minimale d'inflammation du ciel gazeux.

Le brouillard de liquide permet d'abaisser la teneur en oxygène du ciel gazeux par dissolution dans des gouttelettes de ce liquide d'au moins une partie de l'oxygène résiduel contenu dans le gaz sortant du milieu réactionnel. La teneur en oxygène peut ainsi rester en deçà des conditions requises pour qu'il y ait un risque d'explosion. Il convient qu'une molécule gazeuse d'intérêt ne soit pas elle aussi piégée par le brouillard de liquide. Par exemple, supposons que la molécule d'intérêt est de l'Isobutène. Cette molécule est peu soluble dans l'eau. Par conséquent, un brouillard d'eau, ou à base de l'eau, piègera préférentiellement l'oxygène dans le ciel gazeux dans une mesure significativement plus importante que l'Isobutène.

La neutralisation du caractère explosif du ciel gazeux du bioréacteur peut donc être obtenue sur la base de trois effets conjugués : premièrement, la dilution apportée par l'introduction d'un volume inerte constitué par le brouillard de liquide; deuxièmement l'hygrométrie du ciel gazeux, et troisièmement la diminution de la quantité d'oxygène contenue dans le ciel gazeux par solubilisation dans le brouillard de liquide d'une partie de l'oxygène non dissoute lors de la traversée du milieu réactionnel.

En outre, la réduction, voire même l'élimination, du risque d'explosion au moyen d'un brouillard de liquide peut être réalisée en utilisant de faibles quantités de liquide. Par exemple, le brouillard peut être généré avec 0,5 à 1 litre (L) de liquide pour un mètre cube (m³) de ciel gazeux. Ainsi, la quantité de liquide requise peut être extraite, au moins en partie, d'un mout de fermentation utilisé dans un bioprocédé mis en œuvre.

Contrairement à l'état de la technique antérieure divulgué dans le document cité dans ce qui précède, l'invention qui fait l'objet de la présente demande fonctionne aussi pour les fermentations aérobies. Cela est dû au fait que la consommation de liquide pour générer le brouillard est suffisamment faible de sorte qu'il n'y a pas de dilution significative du milieu réactionnel.

A des fins d'illustration, quelques modes de réalisation de l'invention sont décrits en détail en référence aux dessins annexés. Dans cette description, des caractéristiques supplémentaires seront présentées et leurs avantages apparaîtront.

### DESCRIPTION SOMMAIRE DES DESSINS

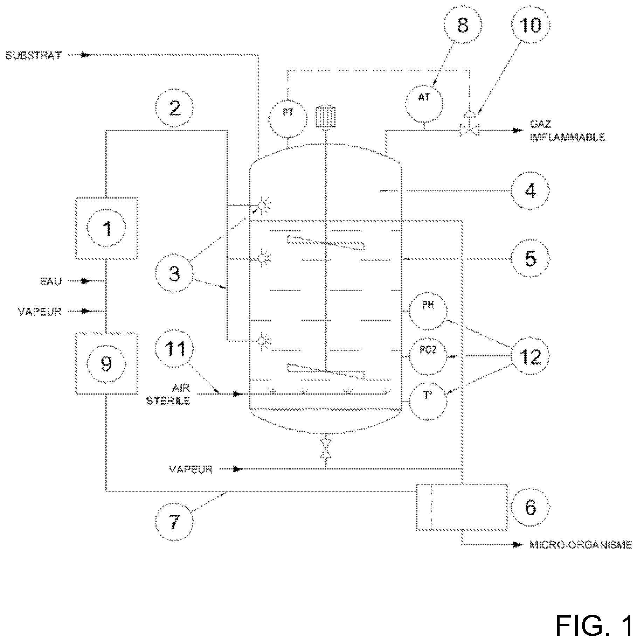

- La figure 1 est un diagramme schématique d'un système de production de biogaz.

### DESCRIPTION DE QUELQUES MODES DE REALISATION

La figure 1 illustre schématiquement un système de production de biogaz. Cette figure présente un diagramme schématique du système de production de biogaz. Le système de production de biogaz comprend un bioréacteur 5 de type fermenteur. Le bioréacteur sera désigné le fermenteur 5 dans ce qui suit à titre d'illustration.

En mode opérationnelle, le fermenteur 5 comprend un milieu réactionnel sous la forme d'un moût de fermentation. Le mout de fermentation peut comprendre un microorganisme associé à un métabolisme microbien. Pour une fermentation aérobie, le fermenteur 5 est traversé par un flux de gaz suffisamment riche en oxygène pour apporter au métabolisme microbien du microorganisme l'oxygène nécessaire à des réactions d'oxydation. Un biogaz et l'oxygène non consommé par le métabolisme microbien sortent du moût de fermentation et occupe un ciel gazeux 4 dans une partie supérieure du fermenteur 5.

Le ciel gazeux 4 du fermenteur comprend un carburant, à savoir un biogaz sortant du mout de fermentation, et un comburant, à savoir de l'oxygène (O₂). La concentration du carburant, le biogaz, dans le comburant, l'oxygène, est un facteur déterminant pour savoir si une combustion peut avoir lieu. La concentration minimale autorisant la combustion constitue un point de départ d'une explosion. Cette concentration constitue une « Limite Inférieure d'Explosivité » (LIE). Une concentration maximale constitue une « Limite Supérieure d'Explosivité » (LSE). La zone délimitée par la LIE et la LSE est le « domaine » ou la « plage » d'explosivité. Le risque d'explosion existe dans le domaine d'explosivité.

Le système de production de biogaz comprend un dispositif de prévention du risque d'explosion du ciel gazeux 4 dans le fermenteur 5. Le dispositif de prévention du risque d'explosion est essentiellement un dispositif de brumisation. Dans le mode de réalisation illustré à la figure 1, le dispositif de brumisation comprend un dispositif de stérilisation 1, un circuit d'alimentation 2 de buses, un ensemble de buses 3 apte à générer un brouillard de liquide, notamment un brouillard d'eau, un dispositif de séparation 6 apte à extraire des microorganismes du moût de fermentation, une boucle de recyclage 7 du moût de fermentation, un dispositif d'analyse 8 de la composition des gaz sortant du fermenteur 5, un dispositif de mise en pression 9 d'un liquide extrait du moût de fermentation, un dispositif de régulation 10 de la pression de ciel gazeux 4, un dispositif de balayage / aération 11 du fermenteur 5.

Le système de production de biogaz peut en outre comprendre un ensemble de capteurs 12. L'ensemble de capteurs 12 permet de suivre une réaction biologique dans le fermenteur 5. L'ensemble de capteurs 12 n'est pas indispensable à un bon fonctionnement du dispositif de prévention du risque d'explosion.

Le dispositif de brumisation fonctionne essentiellement comme suit, Le dispositif de brumisation génère un brouillard de liquide non-inflammable dans le ciel gazeux 4 du bioréacteur par brumisation, Ce brouillard comprend des gouttelettes ayant un diamètre nominal inférieur à 1 mm de sorte que le brouillard de liquide non-inflammable est apte à absorber un rayonnement thermique et à piéger de l'oxygène.

Le dispositif de brumisation peut fonctionner en continu lorsque le fermenteur 5 est en exploitation. Ce dispositif peut fonctionner en conditions stériles, Le dispositif peut aussi être mis en fonctionnement et arrêté sur la base d'une information sur la composition des gaz sortant du fermenteur 5. Cette information peut être comprise dans un signal en provenance du dispositif d'analyse 8,

Le dispositif de brumisation peut être mis en œuvre en phases de fonctionnement établi mais aussi en phases de fonctionnement transitoires. Une phase de fonctionnement transitoire peut comprendre, par exemple, un remplissage du fermenteur 5 ou une vidange du fermenteur 5, Le dispositif de brumisation peut être mis en œuvre lorsque le ciel gazeux 4 du bioréacteur 5 est en pression, par exemple dans une plage de 1 à 4 bars relatifs, ou à pression proche de la pression atmosphérique. Le dispositif peut fonctionner en système clos ou semi ouvert et à pression variable,

Le dispositif de brumisation peut être utilisé lorsque le fermenteur 5 est utilisé en batch ou en Fed batch, avec un apport en substrat continu ou discontinu. Le dispositif peut aussi être utilisé lorsque le fermenteur 5 est utilisé en continu : un apport continu en substrat et une extraction continue du moût de fermentation. Une alimentation continue en substrat avec une élimination en continu d'un volume de moût excédentaire permet de travailler à volume constant dans le fermenteur 5. Le dispositif peut fonctionner en présence d'un balayage du milieu réactionnel. Ce balayage, qui est effectué par le dispositif de balayage / aération 11 illustré à la figure 1, peut être à débit variable, par exemple, un débit compris entre 0 à 1,5 vvm,

Le dispositif de brumisation peut être alimenté en eau ou en autre liquide approprié. Le liquide peut être sous la forme d'une solution d'un agent chimique dans l'eau avec un certain degré de dilution. L'agent chimique peut être, par exemple, un anti-mousse. Le liquide peut provenir, au moins partiellement, d'un recyclage du mout de fermentation. Il convient que le liquide soit inerte tant d'un point de vue chimique et biochimique, que d'un point de vue biologique, afin que le liquide n'ait pas d'impact significatif sur les réactions biologiques souhaitées au sein du fermenteur 5.

Le dispositif de brumisation est généralement peu consommateur en liquide afin de ne pas perturber le fonctionnement global du fermenteur. Une consommation de liquide peut se situer dans une plage, par exemple, de 0,5 à 1 litre (L) de liquide par mètre cube (m³) de gaz. C'est-à-dire, le brouillard peut être généré avec 0,5 à 1 litre (L) de liquide pour un mètre cube (m³) de ciel gazeux. En effet, il convient d'éviter un effet dilution. A cet égard, il convient de noter qu'une brumisation est faiblement consommatrice en eau comparée à, par exemple, un système de type sprinkler.

Le liquide utilisé pour alimenter le dispositif de brumisation, telle que l'eau, peut être considéré comme perdu dans le cas où aucun recyclage n'est fait. Le dispositif peut aussi être alimenté, au moins en partie, par un liquide en provenance du moût de fermentation. Cela permet un fonctionnement particulièrement économique du système, Dans un tel mode de réalisation, le moût de fermentation peut être traité préalablement par le dispositif de séparation 6 illustré à la figure 1.

Le dispositif de brumisation peut éventuellement comprendre une boucle d'asservissement pour réguler la quantité de liquide appliqué aux buses 3 pour former le brouillard dans le ciel gazeux 4. Un asservissement pourrait se faire, par exemple, sur la base d'une teneur en oxygène (O₂) dans le ciel gazeux 4. En effet, le brouillard de liquide permet d'abaisser la teneur en oxygène dans le ciel gazeux 4 pour sortir du domaine d'explosivité. Un mode de réalisation peut donc être doté d'un analyseur de gaz en ligne qui peut donner en continu, ou en semi-continue, une information sur la teneur en oxygène dans le ciel gazeux du fermenteur, et notamment sur la concentration du carburant, le biogaz, dans le comburant, l'oxygène,

Dans le mode de réalisation illustré à la figure 1, le dispositif de stérilisation 1 effectue une stérilisation du liquide appliqué aux buses 3 pour générer le brouillard de liquide dans le ciel gazeux 4 du fermenteur 5. Cette stérilisation peut se faire, par exemple, thermiquement ou par filtration, ou par une combinaison de ces méthodes. Le circuit d'alimentation 2 de buses est de préférence stérile. Le circuit d'alimentation 2 de buses peut être stérilisé en place, par exemple, par de la vapeur d'eau à une température d'environ 121°C avec une durée de stérilisation de 15 à 30 minutes La boucle de recyclage 7 est sensiblement exempte de microorganismes, Un défaut de stérilisation aura pour conséquence une rupture de conditions de fermentation stérile, et donc une contamination, ce qui entrainera généralement une perte de productivité.

Dans ce mode de réalisation, le dispositif de mise en pression 9 permet au liquide d'être pulvérisés sous forme de brouillard au travers les buses 3, Le dispositif de mise en pression 9 peut être localisé avant ou après le dispositif de stérilisation 1. Le dispositif de mise en pression 9 peut s'apparenter à, par exemple, un système par brouillard d'eau basse pression, typiquement inférieure à 12,5 bar, ou à un système par brouillard d'eau haute pression, typiquement supérieur à 35 bar.

Les buses 3 sont dimensionnées pour assurer une taille appropriée de gouttelettes formant le brouillard. La taille des gouttelettes peut aussi dépendre de la pression du liquide et une ou plusieurs caractéristiques structurelles des buses 3 Les buses peuvent être réparties sur la partie supérieure du bioréacteur 5 formée, par exemple, par un dôme et une hauteur de virole, Les buses 3 peuvent être alimentées sélectivement en fonction d'un type de prévention recherchée.

Concernant la taille appropriée de gouttelettes formant le brouillard, les gouttelettes auront typiquement un diamètre inférieur à 1 mm. Il existe une surface d'échange entre un gaz sortant d'un milieu réactionnel et le liquide pulvérisé dans le ciel gazeux. Plus les gouttelettes seront petites, plus importante sera cette surface d'échange, ce qui est favorable à une diminution de l'oxygène dans le ciel gazeux. Les gouttelettes ont avantageusement un diamètre inférieur à 400 µm pour 90 % du volume de liquide injecté. Le brouillard de liquide peut avoir une densité se situant, par exemple, dans une plage de 0,03 à 3 gramme (g) de liquide par mètre cube (m³) de gaz.

Concernant la température du liquide appliqué aux buses 3 pour former le brouillard, il convient d'éviter un gradient de température important à l'interface entre le ciel gazeux 4 et le milieu réactionnel. Il convient donc que la température du liquide soit proche d'une température optimale de réaction dans le milieu réactionnel. Cela évite que les réactions se déroulant dans le milieu réactionnel soient significativement perturbées.

Un mélange gazeux sortant du fermenteur 5, qui comprend le biogaz et le brouillard de liquide, peut subir une filtration afin d'éviter une dissémination d'un microorganisme provenant du fermenteur 5 dans le milieu ambiant. Dans ce cas, il convient d'éviter une condensation avant la filtration du mélange gazeux, Un tel mode de réalisation peut comprendre une jaquette de réchauffage en sortie du fermenteur 5 avant la filtration. Donc, en fonction du type de fermentation, le système de production de biogaz peut comprendre, ou pas, une telle jaquette de réchauffage en sortie du fermenteur.5. Le mélange gazeux sortant du fermenteur 5 peut subir un traitement de type cyclonage destiné à limiter le pourcentage de liquide dans le mélange gazeux à destination d'un éventuel traitement de purification du biogaz produit.

### NOTES

Les modes de réalisation décrits dans ce qui précède en référence aux dessins sont présentés à titre d'illustration. L'invention peut être mise en œuvre de nombreuses façons différentes. Afin d'illustrer cela, quelques alternatives sont indiquées sommairement.

L'invention peut être mise en œuvre dans de nombreux types de produits et procédés dans le domaine de la production du biogaz. Un dispositif de brumisation conforme à l'invention peut être mis en œuvre que l'on soit en condition stérile, dans le cas d'une fermentation, ou en condition non stérile, dans le cas d'une biocatalyse, par exemple.

De façon globale, le terme « bioréacteur » doit donc être interprété de façon large. Ce terme embrasse toute entité dans laquelle une réaction produisant du biogaz peut avoir lieu, telle qu'un fermenteur et un réacteur enzymatiques.

Les remarques qui précèdent montrent que la description détaillée en référence aux figures, illustre l'invention plutôt qu'elle ne la limite. Les signes de références n'ont aucun caractère limitatif. Les verbes « comprendre » et « comporter » n'excluent pas la présence d'autres éléments ou d'autres étapes que ceux listés dans les revendications. Le mot « un » ou « une » précédant un élément ou une étape n'exclu pas la présence d'une pluralité de tels éléments ou de telles étapes.

## Revendications

1. Système de production de biogaz comprenant un bioréacteur (5) apte à comprendre un milieu réactionnel et un ciel gazeux (4) au-dessus du milieu réactionnel, **caractérisé en ce que** le système comprend un dispositif de brumisation (2, 3, 9) apte à générer un brouillard de liquide non-inflammable dans le ciel gazeux du bioréacteur, le brouillard de liquide non-inflammable comprenant des gouttelettes ayant un diamètre nominal inférieur à 1 mm de sorte que le brouillard de liquide non-inflammable est apte à absorber un rayonnement thermique et à piéger de l'oxygène.

2. Système de production de biogaz selon la revendication 1, dans lequel le dispositif de brumisation (2, 3, 9) est apte à produire des gouttelettes ayant un diamètre inférieur à 400 µm pour 90 % du brouillard de liquide non-inflammable.

3. Système de production de biogaz selon l'une quelconque des revendications 1 et 2, dans lequel le liquide non-inflammable formant le brouillard comprend de l'eau.

4. Système de production de biogaz selon l'une quelconque des revendications 1 à 3, dans lequel le dispositif de brumisation (2, 3, 9) est apte à générer le brouillard de liquide non-inflammable avec 0,5 à 1 litre de liquide pour un mètre cube dans le ciel gazeux (4).

5. Système de production de biogaz selon l'une quelconque des revendications 1 à 4, dans lequel le dispositif de brumisation (2, 3, 9) comprend un ensemble de buses (3) reparties sur une partie supérieure du bioréacteur (5), un dispositif de mise en pression (9) apte à mettre en pression un liquide non-inflammable, et un circuit d'alimentation (2) apte à appliquer le liquide non-inflammable mis en pression à l'ensemble de buses (3).

6. Système de production de biogaz selon la revendication 5, comprenant un dispositif de stérilisation (1) apte à effectuer une stérilisation du liquide non-inflammable appliqué à l'ensemble de buses (3).

7. Système de production de biogaz selon l'une quelconque des revendications 5 et 6, comprenant une boucle de recyclage (6, 7) apte à extraire au moins une partie du liquide non-inflammable du milieu réactionnel.

8. Système de production de biogaz selon l'une quelconque des revendications 1 à 7, comprenant un analyseur (8) apte à évaluer si un risque d'explosion existe dans le ciel gazeux (4), le système de production de biogaz étant apte à faire fonctionner le dispositif de brumisation (2, 3, 9) lorsque le d'explosion existe.

9. Procédé de production de biogaz au moyen d'un bioréacteur (5) comprenant un milieu réactionnel et un ciel gazeux (4) au-dessus du milieu réactionnel, **caractérisé en ce qu'**un brouillard de liquide non-inflammable est généré dans le ciel gazeux du bioréacteur par brumisation, le brouillard de liquide non-inflammable comprenant des gouttelettes ayant un diamètre nominal inférieur à 1 mm de sorte que le brouillard de liquide non-inflammable est apte à absorber un rayonnement thermique et à piéger de l'oxygène.

10. Procédé de production de biogaz selon la revendication 9, dans lequel les gouttelettes ont un diamètre inférieur à 400 µm pour 90 % du brouillard de liquide non-inflammable généré.

11. Procédé de production de biogaz selon l'une quelconque des revendications 9 et 10, dans lequel le liquide non inflammable formant le brouillard comprend de l'eau.

12. Procédé de production de biogaz selon l'une quelconque des revendications 9 à 11, dans lequel le brouillard de liquide est généré avec 0,5 à 1 litre de liquide pour un mètre cube dans le ciel gazeux (4).

## Patentansprüche

1. System zur Herstellung von Biogas, das einen Bioreaktor (5) umfasst, der geeignet ist, ein Reaktionsmedium und einen Gasraum (4) über dem Reaktionsmedium zu umfassen, **dadurch gekennzeichnet, dass** das System eine Vernebelungsvorrichtung (2, 3, 9) umfasst, die geeignet ist, einen nicht entflammbaren Flüssigkeitsnebel im Gasraum des Bioreaktors zu erzeugen, wobei der nicht entflammbare Flüssigkeitsnebel Tröpfchen umfasst, die einen Nenndurchmesser von weniger als 1 mm aufweisen, sodass der nicht entflammbare Flüssigkeitsnebel geeignet ist, eine Wärmestrahlung zu absorbieren und Sauerstoff zu fangen.

2. System zur Herstellung von Biogas nach Anspruch 1, wobei die Vernebelungsvorrichtung (2, 3, 9) geeignet ist, Tröpfchen herzustellen, die bei 90 % des nicht entflammbaren Flüssigkeitsnebels einen Durchmesser von weniger als 400 µm aufweisen.

3. System zur Herstellung von Biogas nach einem der Ansprüche 1 und 2, wobei die nicht entflammbare Flüssigkeit, die den Nebel bildet, Wasser umfasst.

4. System zur Herstellung von Biogas nach einem der Ansprüche 1 bis 3, wobei die Vernebelungsvorrichtung (2, 3, 9) geeignet ist, den nicht entflammbaren Flüssigkeitsnebel mit 0,5 bis 1 Liter Flüssigkeit pro einem Kubikmeter im Gasraum (4) zu erzeugen.

5. System zur Herstellung von Biogas nach einem der Ansprüche 1 bis 4, wobei die Vernebelungsvorrichtung (2, 3, 9) einen Satz Düsen (3), die an einem oberen Teil des Bioreaktors (5) verteilt sind, eine Druckbeaufschlagungsvorrichtung (9), die geeignet ist, eine nicht entflammbare Flüssigkeit mit Druck zu beaufschlagen, und einen Speisekreis (2) umfasst, der geeignet ist, die mit Druck beaufschlage nicht entflammbare Flüssigkeit an den Satz Düsen (3) anzulegen.

6. System zur Herstellung von Biogas nach Anspruch 5, das eine Sterilisiervorrichtung (1) umfasst, die geeignet ist, eine Sterilisierung der nicht entflammbaren Flüssigkeit, die an den Satz Düsen (3) angelegt wird, vorzunehmen.

7. System zur Herstellung von Biogas nach einem der Ansprüche 5 und 6, das eine Rückführschleife (6, 7) umfasst, die geeignet ist, mindestens einen Teil der nicht entflammbaren Flüssigkeit aus dem Reaktionsmedium zu extrahieren.

8. System zur Herstellung von Biogas nach einem der Ansprüche 1 bis 7, das einen Analysator (8) umfasst, der geeignet ist, zu beurteilen, ob ein Explosionsrisiko im Gasraum (4) besteht, wobei das System zur Herstellung von Biogas geeignet ist, die Vernebelungsvorrichtung (2, 3, 9) in Gang zu setzen, wenn das Explosionsrisiko besteht.

9. Verfahren zur Herstellung von Biogas mittels eines Bioreaktors (5), der ein Reaktionsmedium und einen Gasraum (4) über dem Reaktionsmedium umfasst, **dadurch gekennzeichnet, dass** im Gasraum des Bioreaktors durch Vernebelung ein nicht entflammbarer Flüssigkeitsnebel erzeugt wird, wobei der nicht entflammbare Flüssigkeitsnebel Tröpfchen umfasst, die einen Nenndurchmesser von weniger als 1 mm aufweisen, sodass der nicht entflammbare Flüssigkeitsnebel geeignet ist, eine Wärmestrahlung zu absorbieren und Sauerstoff zu fangen.

10. Verfahren zur Herstellung von Biogas nach Anspruch 9, wobei die Tröpfchen bei 90 % des erzeugten nicht entflammbaren Flüssigkeitsnebels einen Durchmesser von weniger als 400 µm aufweisen.

11. Verfahren zur Herstellung von Biogas nach einem der Ansprüche 9 und 10, wobei die nicht entflammbare Flüssigkeit, die den Nebel bildet, Wasser umfasst.

12. Verfahren zur Herstellung von Biogas nach einem der Ansprüche 9 bis 11, wobei der Flüssigkeitsnebel mit 0,5 bis 1 Liter Flüssigkeit pro einem Kubikmeter im Gasraum (4) erzeugt wird.

## Claims

1. A system for producing biogas comprising a bioreactor (5) adapted to comprise a reaction medium and a gas blanket (4) above the reaction medium, **characterised in that** the system comprises a misting device (2, 3, 9) adapted to generate a mist of non-flammable liquid in the gas blanket of the bioreactor, the mist of non-flammable liquid comprising droplets having a nominal diameter less than 1 mm such that the mist of non-flammable liquid is able to absorb a thermal radiation and trap oxygen.

2. A system for producing biogas according to claim 1, wherein the misting device (2, 3, 9) is adapted to produce droplets that have a diameter less than 400 µm for 90% of the mist of non-flammable liquid.

3. A system for producing biogas according to any of claims 1 and 2, wherein the non-flammable liquid forming the mist comprises water.

4. A system for producing biogas according to any of claims 1 to 3, wherein the misting device (2, 3, 9) is adapted to generate the mist of non-flammable liquid with 0.5 to 1 litre of liquid for one cubic metre in the gas blanket (4).

5. A system for producing biogas according to any of claims 1 to 4, wherein the misting device (2, 3, 9) comprises a set of nozzles (3) distributed over an upper portion of the bioreactor (5), a device for pressurising (9) adapted to pressurise a non-flammable liquid, and a feed circuit (2) adapted to apply the pressurised non-flammable liquid to the set of nozzles (3).

6. A system for producing biogas according to claim 5, comprising a device for sterilising (1) adapted to carry out a sterilisation of the non-flammable liquid applied to the set of nozzles (3).

7. A system for producing biogas according to any of claims 5 and 6, comprising a recycling loop (6, 7) adapted to extract at least one portion of the non-flammable liquid from the reaction medium.

8. A system for producing biogas according to any of claims 1 to 7, comprising an analyser (8) adapted to evaluate if a risk of explosion exists in the gas blanket (4), the system for producing biogas being adapted to make the misting device (2, 3, 9) operate when the risk of explosion exists.

9. A method for producing biogas by means of a bioreactor (5) comprising a reaction medium and a gas blanket (4) above the reaction medium, **characterised in that** a mist of non-flammable liquid is generated in the gas blanket of the bioreactor by misting, the mist of non-flammable liquid comprising droplets having a nominal diameter less than 1 mm such that the mist of non-flammable liquid is able to absorb a thermal radiation and trap oxygen.

10. A method for producing biogas according to claim 9, wherein the droplets have a diameter less than 400 µm for 90% of the mist of non-flammable liquid generated.

11. A method for producing biogas according to any of claims 9 and 10, wherein the non-flammable liquid forming the mist comprises water.

12. A method for producing biogas according to any of claims 9 to 11, wherein the mist of liquid is generated with 0.5 to 1 litre of liquid for one cubic metre in the gas blanket (4).
